# EUROPÄISCHE PATENTANMELDUNG

(11) **EP 0 987 046 A1**
(43) Veröffentlichungstag der Anmeldung: **22.03.2000**
(21) Anmeldenummer: 99118213.0
(22) Anmeldetag: 14.09.1999
(51) Int. Cl.: A61M 39/10

(54) **Steckverbindung für Infusionsleitungen**

(30) Priorität: 14.09.1998 DE 19841869
(71) Anmelder: Yilmaz, Memduh, 40699 Erkrath (DE)
(72) Erfinder: Yilmaz, Memduh, 40699 Erkrath (DE)
(74) Vertreter: COHAUSZ HANNIG DAWIDOWICZ & PARTNER

(57) **Zusammenfassung**

System zur Verbindung und/oder zum Verschluß von Infusionsleitungen mit zu Luer-Lock-Verbindungen kompatibelen "male"-Adaptern, die einen Anschlußstutzen aufweisen, und mit entsprechenden "female"-Adaptern, die jeweils eine den Anschlußstutzen aufnehmende Muffe aufweisen, wobei der female-Adapter 2 eine Schutzkappe 10 in Form einer die Muffe 5 umgebenden Hülse aufweist, die einen die Muffe 5 umgebenden Ringraum 11 umschließt.

## Beschreibung

Die vorliegende Erfindung betrifft ein System zur Verbindung und/oder zum Verschluß von Infusionsleitungen mit "male"-Adaptern, die einen Anschlußstutzen aufweisen, und mit "female"-Adaptern, die jeweils eine den Anschlußstutzen aufnehmende Muffe aufweisen.

In der Medizin werden intravenöse oder arterielle Katheter und Infusionsleitungen einerseits zur Flüssigkeits- und Medikamententherapie und andererseits zur Überwachung eingesetzt. Bei diesen Systemen wird eine Große Zahl von Zubehörteilen (Infusionszubehör) verwendet, die miteinander entweder durch einfache Steckverbindungen oder üblicherweise durch Steck-Schraubverbindungen (sogenannte Luer-Lock Verbindungen) gekoppelt werden. Dabei läßt sich der Anschlußstutzen des männlichen ("male") Adapters in die Muffe des weiblichen ("female") Adapters dichtend einstecken, wobei die Verbindung durch eine Überwurfmutter gegen ein Herausrutschen gesichert wird. Mit dem bekannten und größtenteils normierten Infusionszubehör werden z.B. intravasale Verweilkatheter, Mehrweghähne, Hahnenbänke, Entnahme- oder Zuspritzkanülen und/oder Infusionsleitungen realisiert und zu leistungsfähigen Systemen kombiniert.

Nachteil der bislang bekannten Systeme ist, daß die female-Adapter insofern kontaminationsgefährdet sind, als der Rand der Muffen frei zugänglich ist und in direkter Verbindung mit den Infusionsleitungen steht. Im nicht konnektierten Zustand besteht somit die Gefahr der Kontamination der Leitung über eine Berührung des Randes. Eine Berührung geschieht dabei oft beim Zusammenstecken der einzelnen Verbindungsstücke oder beim Wechseln der einzelnen Zubehörteile. Der Adapter kommt außerdem leicht in Kontakt mit der nicht sterilen Haut des Patienten oder mit dem Bettlaken. Zwar ist es bekannt, die Muffe mit einem Verschlußstopfen zu verschließen, um die Kontaminationsgefahr zu verringern. Dies bedeutet jedoch einen zusätzlichen Arbeitsschritt, der häufig vergessen wird. Das Problem der Kontamination wird dadurch verstärkt, daß der female-Adapter meist das proximale Endstück der Infusionsleitung ist und somit in andauerndem Kontakt zum Patienten steht.

Im Falle des male-Adapters ist die Gefahr der Kontamination nicht so groß, da der Anschlußstutzen zum Großteil von der Überwurfmutter abgedeckt ist. Lediglich der äußere Rand des Anschlußstutzen überragt die Überwurfmutter und ist der Kontaminationsgefahr ausgesetzt.

Aufgabe der vorliegenden Erfindung ist es, ein System zur Vebindung und/oder zum Verschließen von Infusionsleitungen zu schaffen, das kostengünstig ist und bei einfachem Aufbau der male- bzw. female-Adapter eine kontaminationsfreie Handhabung der einzelnen Teile gewährleistet.

Diese Aufgabe wird durch ein System nach Anspruch 1 gelöst.

Besonderes Merkmal des erfindungsgemäßen Systems ist, daß insbesondere der female-Adapter mit einer Schutzkappe versehen ist, welche die Muffe umschließt. Diese Schutzkappe bildet eine glockenförmige Ummantelung, die wegen des zwischen Ihr und der Muffe verbleibenden Ringraumes nicht im direkten Berührungskontakt mit dem Rand der Muffe steht. Dadurch, daß bei der Handhabung der Adapter lediglich die Ummantelung den Berührungen mit der Haut des Patienten, dem Bettlaken oder den Händen des Personals ausgesetzt ist, kann die Gefahr der Kontamination des Muffenrandes verringert werden. Die Schutzkappe wirkt somit als beabstandeter Schild und als Wegverlängerung gegen das Eindringen unerwünschter Keime.

Besonders vorteilhaft an dem erfindungsgemäßen System ist, daß die Gefahr der Kontamination mit einfachsten Mittel drastisch verringert werden kann. Die bekannten Systeme, insbesondere die Luer-Lock-Verbindungen, lassen sich ohne großen Aufwand und auf kostengünstige Weise mit Schutzkappen ausrüsten, die im einfachsten Falle wie Kragen oder Glocken derart an die Muffen angespritzt werden, daß ihre Öffnung zum male-Adapter hin offen ist. Dabei ist die Handhabung des Systems einfach und erfordert keine besonderen zusätzlichen Arbeitsschritte des Behandlungspersonals.

Die Sicherung der Verbindung geschieht vorteilhafterweise dadurch, daß eine Überwurfmutter, die den male-Adapter (Verbindungsstück) umgibt, mittels eines Schraub- oder Bajonettverschlusses am female-Adapter befestigt wird. Vorteilhafterweise sind die Überwurfmutter und der male-Adapter derart ausgebildet, daß die Mutter am Außenumfang der Muffe angreift und in den freien Ringraum unter der Schutzkappe einführbar ist. Durch dieses doppelte Ineinanderstecken der Bauteile ist den möglichen Verunreinigungen eine besonders wirksame Sperre entgegengesetzt.

In einer besonders vorteilhaften Ausführungsform des Systems ist die Schutzkappe derart ausgebildet, daß sie den dem male-Adapter zugerichteten Rand der Muffe überragt. Der Rand liegt somit innerhalb des von der Schutzkappe gebildeten Zylinders und ist bei normaler Handhabung völlig vor Berührungskontakt geschützt. Um die Handhabung der Teile zu vereinfachen ist es vorteilhaft, wenn die Adapter insbesondere auch der female-Adapter Griffe, insbesondere in Form zweier gegenüberliegender radial abstehender Flügel, aufweisen. Die Griffe lassen sich gut greifen und gewährleisten eine gute Kraftübertragung auf die Adapter, so daß diese fest gegeneinander verschraubt werden können.

Um die Gefahr der Kontamination weiter zu senken, ist es vorteilhaft, die Schutzkappe so auszubilden, daß sie in axialer Richtung insbesondere zwischen einer vorderen und einer hinteren Grenzstellungen verschieblich auf dem female-Verbindungsstück gelagert ist. Dadurch wird einerseits eine effektive Bedeckung der Kupplungsstelle und andererseits eine gute Handhabbarkeit der Zubehörteile gewährleistet. Vorteilhafterweise weist das Verbindungsstück Mittel auf, die die axiale Verschiebung der Schutzkappe durch Kraftschluß oder durch Formschluß begrenzen.

In einer besonders zu bevorzugenden Ausführungsform ist die Schutzkappe so konzipiert, daß sie beim Lösen der Verbindung also beim Herausziehen des male-Adapters bis zu der hinteren Grenzstellung mitgenommen wird und dann einrastet. Damit kann ein sehr tiefes Einliegen der Muffe in der Glocke und damit ein besonders guter Schutz erreicht werden. Beim Zusammenfügen der Adapter wird die Kappe eigenständig zurückgeschoben um die Kontaktierung zu erleichtern. Überhaupt ist es vorteilhaft, die Teile aus insbesondere transparentem Kunststoff zu fertigen, um das Zusammenfügen zu erleichtern und die Funktionstüchtigkeit zu kontrollieren.

Das erfindungsgemäße System weist neben den durchgängigen Verbindungsstücken auch Verschlußstücke als Adapter auf, die mit der Schutzkappe versehen sind. So ist es besonders vorteilhaft, für den Verschluß von male-Verbindungsstücken vorgesehene female-Adapter zu schaffen, die mit der Schutzkappe versehen sind. Die Adapter können mit einem Boden verschlossen sein. Es ist jedoch besonders vorteilhaft, die Verschlußstücke universell auszubilden und einerseits mit einem male- und andererseits mit einem female-Adapter zu versehen, wobei zwischen den beiden Adaptern keine Verbindung besteht. Es ist ebenso möglich, female-Endstücke (Verschlüsse) für male-Adapter mit den erfindungsgemäßen Schutzkappen auszurüsten.

Besonders vorteilhafte Ausführungsformen des erfindungsgemäßen Systems sind in den Figuren dargestellt und werden im folgenden näher beschrieben. Es zeigen:
- **Figur 1:**: ein Paar von Adaptern,
- **Figur 2:**: ein female-Adapter mit verschieblicher Schutzkappe,
- **Figur 3:**: ein female-Adapter mit versenkbarem Griffteil und
- **Figur 4:**: ein Kombi-Verschlußstück

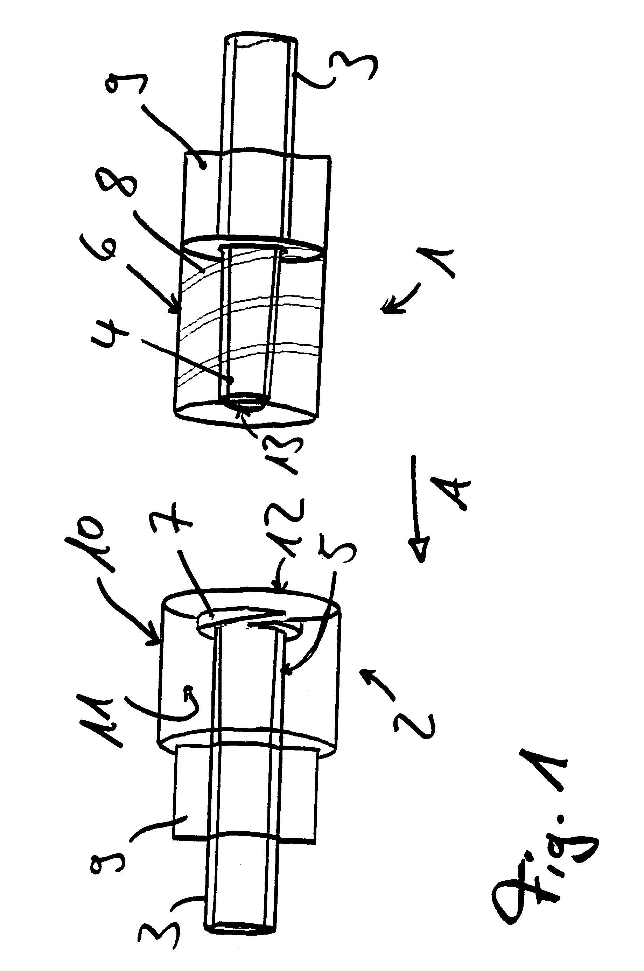

In Figur 1 sind ein "male"-Adapter 1 und ein "female"-Adapter 2 eines Systems zur Verbindung von Infusionsleitungen 3 in der Art einer Luer-Lock Verbindung dargestellt. Die Adapter sind in diesem Falle aus Kunststoff gespritzte Verbindungsstücke, die im verbundenen Zustand den Durchfluß durch die Infusionsleitung sichern. Dabei weist der "male"-Adapter 1 einen leicht konisch zulaufenden Anschlußstutzen 4 auf, der in eine entsprechende Muffe 5 des "female"-Adapters 2 in Pfeilrichtung A dichtend einschiebbar ist. Nachdem der male-Adapter 1 in die Muffe 5 eingeschoben ist, wird die Verbindung bekanntermaßen mittels einer den male-Adapter 1 umgebenden Flügelmutter 6 verschraubt, wobei die Flügelmutter 6 an einem den Rand der Muffe 5 umgebenden Außengewinde 7 angreift und über ein Innengewinde 8 aufschraubbar ist. Zum komfortablen Halt weisen beide Adapter 1 und 2 Flügel 9 auf, die ein Griffteil bilden.

Erfindungsgemäß ist die Muffe 5 von einer Schutzkappe 10 in Form einer die Muffe 5 umgebenden glockenförmigen Hülse umgeben, die an der zum male-Adapter 1 gerichteten Seite offen ist. In diesem Falle ist die Schutzkappe 10 an den female-Adapter angespritzt. Die Schutzkappe 10 umschließt einen freien Ringraum 11 in den die Überwurfmutter 6 einführbar ist. Die Schutzkappe 10 überragt mit ihrem Rand 12 die Stirnseite der Muffe 5. Im Falle des male-Adapters 1 überragt die Überwurfmutter 6 die Stirnfläche 13 des Anschlußstutzens 4.

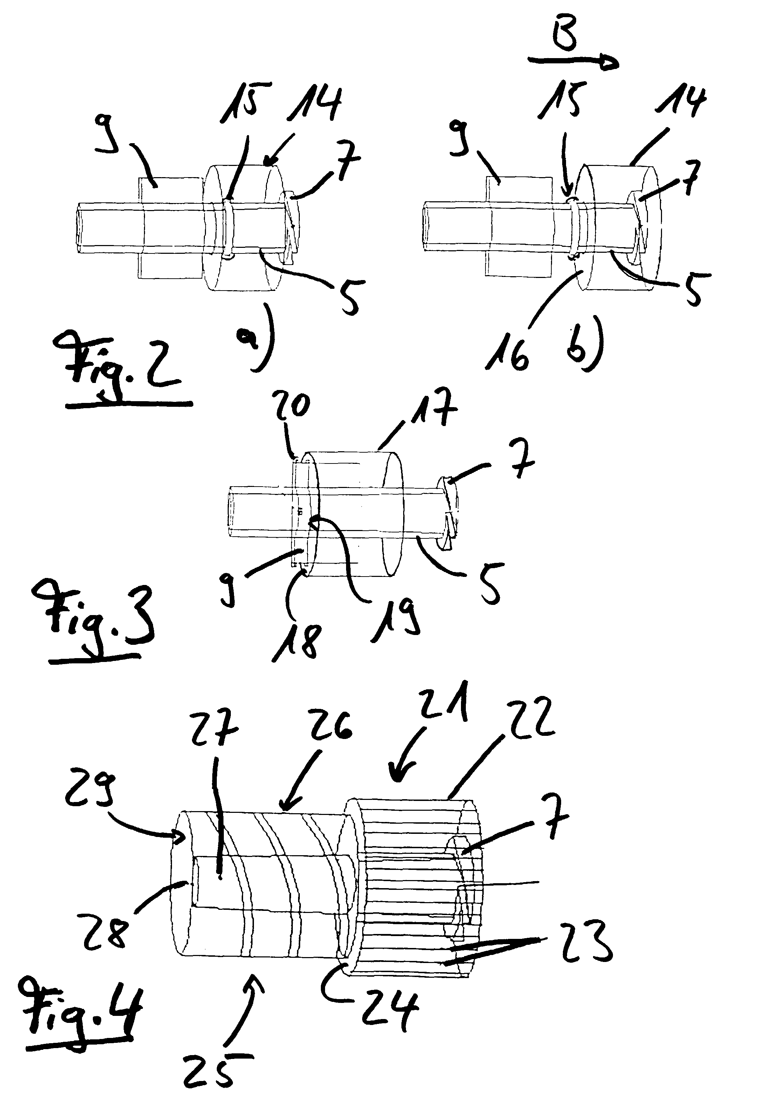

In der Figur 2 ist ein female-Adapter dargestellt, dessen Schutzkappe 14 in axialer Richtung verschieblich ist. In der in Figur 2a dargestellten Position ist die Schutzkappe 14 in der hinteren durch den Flügel 9 begrenzten Anschlagposition, in der das Gewinde 7 der Muffe 5 freiliegt. Die Bewegung der Schutzkappe 14 wird durch einen Ring 15 gehemmt, der die Muffe 5 umgibt. Die Bewegung der Schutzkappe 14 kann durch eine Feder unterstützt werden. In der anderen Anschlagposition (Figur 2b) ist die Schutzkappe 14 in Richtung des Pfeiles B nach vorne verschoben und bedeckt den Rand der Muffe 5 vollständig. Der Ring 15, der nun außerhalb des Bodens 16 der Schutzkappe 14 zu liegen kommt, sichert deren Lage in dieser Position.

Figur 3 zeigt eine Schutzkappe 17, die über die Flügel 9 hinweg veschieblich gelagert ist. Dazu weist die Schutzkappe 17 in ihrem Boden 18 einen Schlitz 19 auf, in dem die Flügel eintauchen. In diesem Zustand liegt die Muffe 5 mit dem Gewinde 7 völlig frei. Mit diesen Schutzkappen können die bekannten Luer-Lock-Verbindungen ausgestattet werden. In diesem Fall weisen die Flügel 9 kleine Vorsprünge 20 auf, die eine Grenzstellung für die Schutzkappe 17 definieren.

In Figur 4 ist ein Kombi-Verschlußstück gezeigt, das einen als Verschluß ausgebildeten female-Adapter 21 mit fester Schutzkappe 22 aufweist. Die Oberfläche der Schutzkappe 22 ist in der Art einer Rändelschraube mit Riefen 23 versehen, die einen sicheren Griff gewährleisten. Die Schutzkappe 22 bedeckt das Gewinde 7 vollständig. Der Boden 24 der Schutzkappe 22 ist geschlossen, so daß ein Durchfluß verhindert ist. In axialer Verlängerung des female-Adapters 21 ist ein male-Adapter 25 angeordnet, der als Verschluß für female-Adapter dient. Entsprechend der vorher dargestellten Ausführungsformen weist der male-Adapter 25 eine Überwurfmutter 26 auf, die einen Anschlußstutzen 27 und dessen Stirnfläche 28 unter Ausbildung eines Ringraumes 29 vollständig abdeckt.

## Patentansprüche

1. System zur Verbindung und/oder zum Verschluß von Infusionsleitungen mit "male"-Adaptern, die einen Anschlußstutzen aufweisen, und mit "female"-Adaptern, die jeweils eine den Anschlußstutzen aufnehmende Muffe aufweisen,
**dadurch gekennzeichnet,** daß der female-Adapter (2) eine Schutzkappe (10) in Form einer die Muffe (5) umgebenden Hülse aufweist, die einen die Muffe (5) umgebenden Ringraum (11) umschließt.

2. System nach Anspruch 1,
**dadurch gekennzeichnet**, daß die Schutzkappe (10) den dem male-Adapter (1) zugerichteten Rand der Muffe (5) überragt.

3. System nach Anspruch 1 oder 2,
**dadurch gekennzeichnet,** daß der male-Adapter (1) eine Überwurfmutter (6) eines Schraub- oder Bajonettverschlusses aufweist, die am Außenumfang der Muffe (5) angreift und die in den freien Ringraum (11) einführbar ist.

4. System nach einem der vorherigen Ansprüche,
**dadurch gekennzeichnet,** daß der female-Adapter (2) einen Griff, insbesondere in Form zweier gegenüberliegender radial abstehender Flügel (9) aufweist.

5. System nach einem der vorherigen Ansprüche,
**dadurch gekennzeichnet**, daß die Schutzkappe (10) in axialer Richtung verschieblich auf dem female-Adapter (2) gelagert ist.

6. System nach einem der vorherigen Ansprüche,
**dadurch gekennzeichnet**, daß die Schutzkappe (10) ein Rastmittel, insbesondere in Form eines die Muffe (5) umgebenden Kragens (15) aufweist, das in einer Nut einrastbar ist.

7. System nach einem der vorherigen Ansprüche,
**dadurch gekennzeichnet,** daß der female-Adapter (2) ein Mittel aufweist, das die axiale Verschiebung der Schutzkappe (10) durch Kraftschluß oder durch Formschluß begrenzt.

8. System nach einem der vorherigen Ansprüche,
**dadurch gekennzeichnet**, daß die Schutzkappe (10) über den Griff (9) hinweg verschieblich ist.

9. System nach einem der vorherigen Ansprüche,
**dadurch gekennzeichnet**, daß die Schutzkappe (10) aus insbesondere transparentem Kunststoff gefertigt ist.

10. System nach einem der vorherigen Ansprüche,
**dadurch gekennzeichnet,** daß die Überwurfmutter (6) derart am male-Adapter (1) drehbar fixiert ist, daß sie den Rand (13) des Anschlußstutzens (4) überragt.

11. System nach einem der vorherigen Ansprüche,
**dadurch gekennzeichnet**, daß die Schutzkappe (10) an den female-Adapter (2) insbesondere an den Griff (9) angespritzt ist.

12. System nach einem der vorherigen Ansprüche,
**dadurch gekennzeichnet**, daß die Adapter (1,2) kompatibel zu Luer-Lock-Verbindungen sind.

13. System nach einem der vorherigen Ansprüche,
**gekennzeichnet durch** einen zweigeschlechtlichen Adapter (21,25) zum Verschluß einer Leitung, der einerseits eine Muffe mit umgebender Schutkappe (22) und andererseits einen Anschlußstutzen (27) mit umgebender Überwurfmutter (26) aufweist.
